(19) **Europäisches Patentamt / European Patent Office / Office européen des brevets**

(11) **EP 2 677 298 B1**

(12) **FASCICULE DE BREVET EUROPEEN**

(45) Date de publication et mention de la délivrance du brevet:
**01.02.2017 Bulletin 2017/05**

(51) Int Cl.:
***G01N 19/02*** *(2006.01)* *G01N 33/36* *(2006.01)*

(21) Numéro de dépôt: **13172997.2**

(22) Date de dépôt: **20.06.2013**

(54) **Dispositif et procédé de détermination d'une force de frottement à l'interface entre deux surfaces**

Vorrichtung und Verfahren zum Bestimmen einer Reibungksraft an der Grenzfläche zweier Oberflächen

Device and method for determination of a friction force at the interface between two surfaces.

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.06.2012 FR 1255799**

(43) Date de publication de la demande:
**25.12.2013 Bulletin 2013/52**

(73) Titulaire: **Institut National de Recherche en Sciences et Technologies pour l'Environnement et l'Agriculture (IRSTEA) 92160 Antony (FR)**

(72) Inventeurs:
• **Stoltz, Guillaume 51000 CHÂLONS-EN-CHAMPAGNE (FR)**
• **Gallo, Roland 91700 SAINT GENEVIEVE DES BOIS (FR)**

• **Poulain, Daniel 33920 SAINT SAVIN (FR)**

(74) Mandataire: **Petit, Maxime et al Ipsilon Le Centralis 63, avenue du Général Leclerc 92340 Bourg-la-Reine (FR)**

(56) Documents cités:
**GB-A- 2 455 564 SU-A2- 974 164 US-A- 3 020 744**

• **BRIANÇON L ET AL: "A new procedure for measuring geosynthetic friction with an inclined plane", GEOTEXTILES AND GEOMEMBRANES, ELSEVIER, AMSTERDAM, NL, vol. 29, no. 5, 1 avril 2011 (2011-04-01), pages 472-482, XP028278927, ISSN: 0266-1144, DOI: 10.1016/J.GEOTEXMEM.2011.04.002 [extrait le 2011-04-12]**

**Description**

L'invention est relative à un procédé de détermination d'une force de frottement à l'interface entre deux surfaces qui sont en contact l'une avec l'autre.

Il est connu de déterminer une force de frottement à l'interface entre deux surfaces qui sont en contact l'une avec l'autre. En particulier, il est connu de déterminer des caractéristiques en frottement (par ex. un angle de frottement) de géosynthétiques (géotextiles et produits apparentés, géomembranes et produits apparentés), en contact avec divers sols, en utilisant un appareillage à plan incliné.

La Norme européenne EN ISO 12957-2 :2005 (Norme internationale ISO 12957-2 :2005) décrit une telle méthode de détermination. Selon cette méthode l'angle de frottement d'un complexe sol/géosynthétique ou géosynthétique/géosynthétique est déterminé en mesurant l'angle auquel une boîte remplie de sol glisse sur une base lorsque cette dernière qui supporte le géosynthétique est inclinée à une vitesse constante.

On connaît du document intitulé « Justification d'une modification de la norme relative au frottement des interfaces géosynthétiques au plan incliné » de Briançon et al, Rencontres Géosynthétiques 2011, Tours, France, 219-228, une méthode de détermination de l'angle de frottement d'un complexe sol/géosynthétique ou géosynthétique/géosynthétique. Pour la mise en oeuvre de cette méthode, un géosynthétique est par exemple fixé à la face inférieure d'un boîtier dit supérieur et un autre géosynthétique est fixé sur un plan qui est inclinable à partir de l'horizontale. Le boîtier supérieur repose sur le plan en emprisonnant entre eux les deux géosynthétiques en contact l'un avec l'autre. Le boîtier est lié à une paroi solidaire du plan par l'intermédiaire d'un câble. Au début de la méthode, le plan est dans une position initiale horizontale et le câble servant à retenir le boîtier n'est pas tendu. On augmente ensuite à vitesse constante l'inclinaison du plan à partir de la position initiale et l'on mesure la force retenant le boîtier supérieur lorsque le câble passe d'une position relâchée à une position tendue, c'est-à-dire après que le boîtier ait glissé sur une distance axiale prédéterminée.

Cette méthode n'est toutefois pas satisfaisante dans la mesure où, dans les conditions d'essai décrites :

- la force de frottement ne peut être déterminée entre le départ du glissement et le moment où le câble servant à retenir le boîtier se met en tension;
- le boîtier peut être soumis au début du glissement à un mouvement accéléré; dans ce cas, le boitier est soumis à une force d'accélération qui est en partie compensée dans la tension du câble servant à le retenir, ce qui perturbe la détermination de la force de frottement à l'interface.

On connaît également du document GB 2 455 564 un dispositif de mesure d'un coefficient de frottement entre deux surfaces en contact pouvant être comprimées par l'intermédiaire d'un poids.

Le dispositif comprend un mécanisme de traction pour provoquer le déplacement de la surface supérieure, éventuellement chargée, en contact avec la surface inférieure. Ce dispositif présente toutefois des désavantages puisqu'il ne permet de mesurer un coefficient de frottement qu'au début du glissement (coefficient de frottement statique) mais pas au cours de la suite du mouvement de glissement.

La présente invention prévoit de remédier à au moins un des inconvénients exposés ci-dessus en proposant un procédé de détermination d'une force de frottement à l'interface entre deux surfaces en contact l'une avec l'autre, le procédé mettant en oeuvre un support dont l'inclinaison par rapport à l'horizontale est susceptible de varier au cours du temps et un élément mobile qui est susceptible de glisser sous la seule action de son poids sur le support à partir d'un angle d'inclinaison donné dudit support, ledit élément mobile étant conçu de façon à permettre d'appliquer une charge sur deux surfaces en contact et disposées l'une au dessus de l'autre sur le support, générant ainsi, avec l'inclinaison du support, une force de frottement à l'interface entre les deux surfaces, ledit élément mobile étant lié à un élément de référence par au moins un organe élastique qui est susceptible de se déformer élastiquement selon une direction axiale et qui est apte à exercer une force de retenue sur ledit élément mobile, l'élément de référence ayant une inclinaison qui suit celle du support et une position axiale qui reste fixe quelle que soit l'inclinaison, le procédé comprenant les phases suivantes :

- augmentation de l'angle d'inclinaison dudit support depuis un état initial dans lequel ledit au moins un organe élastique est dans un état élastiquement prédéformé,

- mesure de la force de retenue de l'élément mobile à partir de la déformation élastique dudit au moins un organe élastique,

- détermination d'une force de frottement à l'interface entre les surfaces en fonction de la force de retenue ainsi mesurée.

Lorsque l'angle d'inclinaison du support augmente, pour un angle d'inclinaison donné qui dépend des surfaces en contact, un glissement s'opère à l'interface entre les deux surfaces. L'élément mobile commence à glisser sur le support naturellement, c'est-à-dire sous la seule action de la composante tangentielle de son propre poids (sans l'intervention d'un dispositif ou mécanisme annexe de traction ou de poussée de l'élément mobile). Comme ledit au moins un organe élastique est initialement prédéformé (par exemple prétendu ou précompressé, alors que l'élément mobile n'a pas commencé à glisser et que l'inclinaison du support n'a pas encore été modifiée par rapport à son état initial), il exerce sur l'élément mobile une force de retenue supérieure à celle qui serait exercée si ledit au moins un organe élastique n'était pas initialement prédéformé. L'élément mobile est donc mieux retenu au début du mouvement de glissement et une possible accélération du mouvement de glissement de l'interface entre les deux surfaces est donc fortement réduite.

Pour les mêmes raisons, la force de retenue de l'élément mobile peut être mesurée dès le début du glissement, ce qui n'est pas envisageable dans le premier document de l'art antérieur décrit ci-dessus dans la mesure où le câble n'est pas encore tendu au moment où le boîtier commence à glisser.

Par ailleurs, le procédé selon l'invention permet de mesurer le coefficient de frottement (ou l'angle de frottement) pour des conditions (ou mouvement) de glissement dont la vitesse peut être contrôlée, ce que ne permet pas le dispositif du deuxième document de l'art antérieur, à savoir GB 2 455 564.

L'intérêt d'utiliser un ou plusieurs organes déformables est d'autoriser le mouvement de glissement de l'interface entre les deux surfaces quelle que soit la force nécessaire pour retenir l'élément mobile.

On notera que les deux surfaces (surfaces inférieure et supérieure) sont généralement des surfaces d'échantillons de matériaux dont par exemple l'un au moins est de type géosynthétique et les échantillons prennent, par exemple, la forme de bandes ou de nappes. Le support est par exemple un plan ou une table inclinable et le matériau géosynthétique inférieur est directement en contact avec le plan ou la table tandis que le matériau supérieur (ex : géosynthétique) est disposé sous l'élément mobile. L'une des deux surfaces peut alternativement être la surface d'un sol (ex : sable, mélange incluant du sable, gravier...) en contact avec l'autre surface d'un géosynthétique.

L'élément mobile est conçu pour permettre, de façon directe ou indirecte, l'application d'une charge (ou d'un poids) sur les deux surfaces en contact. Ainsi, l'élément mobile peut être un solide par exemple réalisé en un seul tenant qui repose directement ou indirectement (par exemple via un autre élément tel qu'un autre géosynthétique) sur les éléments dont les deux surfaces sont en contact. Le poids de ce solide est donc appliqué sur les deux surfaces en contact. Alternativement, l'élément mobile est un boîtier creux, ouvert à sa partie inférieure, d'un poids prédéterminé et qui peut être rempli ou non d'un matériau de remplissage d'un poids prédéterminé. Le boîtier peut ainsi reposer directement ou indirectement (par exemple, par l'intermédiaire du matériau de remplissage) sur les éléments dont les deux surfaces sont en contact. Le poids de ce boîtier et/ou de la charge qu'il contient peut donc être appliqué sur les deux surfaces en contact. On notera que le boîtier peut être équipé d'organes de roulement afin que son poids repose directement sur le support inclinable et non sur les surfaces en contact, seul le poids de la charge qu'il contient étant appliqué sur les surfaces en contact.

Selon d'autres caractéristiques prises isolément ou en combinaison l'une avec l'autre :

- ledit au moins un organe élastiquement déformable est prédéformé suivant une contrainte supérieure ou égale à 1 N ;

- l'angle d'inclinaison dans l'état initial dépend de la nature des surfaces en contact (il peut ainsi être d'autant plus élevé que le coefficient de frottement entre les surfaces est élevé) ; il peut donc être adapté afin de rendre le procédé plus performant ; l'angle d'inclinaison dans l'état initial peut donc être sensiblement nul ou non nul (par exemple de l'ordre de quelques degrés) en fonction de la nature des surfaces en contact ; pour des surfaces en contact très frottantes (coefficient de frottement élevé), c'est-à-dire pour lesquelles le début du glissement intervient pour un angle d'inclinaison du support élevé (ex : 30°), l'angle d'inclinaison prédéterminé dans l'état initial peut être d'autant plus élevé (ex : 10°) ; inversement, l'angle d'inclinaison prédéterminé dans l'état initial est d'autant moins élevé, voire nul (surfaces en contact sensiblement à l'horizontale), lorsque les surfaces en contact sont peu frottantes ; dans l'état initial, ledit au moins un organe élastique est déjà dans un état prédéformé; ainsi, pour un certain angle d'inclinaison du support correspondant au départ d'un glissement significatif de l'élément mobile, le ou les organes élastiques se déforment élastiquement à partir de leur état initial prédéformé, limitant par conséquence la mise en mouvement de glissement accéléré de l'élément mobile par comparaison avec le cas où le ou les organes ne seraient pas initialement déformés ; de l'ordre de quelques degrés ;

- ledit au moins un organe élastique est un ressort ou un câble élastique ; leur constante de raideur est choisie de manière adaptée aux conditions de mise en oeuvre du procédé (matériaux, poids de l'élément mobile et de son éventuelle charge aditionnelle...) ;

- le procédé prévoit de mesurer la force de retenue avant que l'élément mobile n'adopte un mouvement de glissement (par exemple uniforme) sur le support à partir d'un angle d'inclinaison donné ;cette phase de mesure préalable au glissement est rendue possible par la mise en déformation préalable du ou des organes élastiques ;

- à partir d'un angle d'inclinaison donné, l'élément mobile adopte un mouvement de glissement (par exemple uniforme) sur le support et ledit au moins un organe élastique se déforme élastiquement au fur et à mesure que l'angle d'inclinaison augmente, la force de retenue étant mesurée durant ledit mouvement de glissement ; on notera que, selon la ou les interfaces testées, le mouvement de glissement peut ne pas être totalement uniforme mais constitué d'une succession de mouvements de type « collé-glissé » ;dans ce cas l'accélération de chaque mouvement unitaire est négligeable et le procédé s'appliquera comme décrit plus haut ;

- l'élément de référence est fixé au support ; cet élément est par exemple une paroi ;

- ledit au moins un organe élastique est prédéformé en étirement et tend à empêcher l'élément mobile de s'éloigner de l'élément de référence ;

- ledit au moins un organe élastique est prédéformé en compression et tend à empêcher l'élément mobile de se rapprocher de l'élément de référence ;

- au moins une des deux surfaces en contact l'une avec l'autre est une surface de type géosynthétique ;

- l'augmentation de l'angle d'inclinaison dudit support est effectuée avec une vitesse variable ; ceci permet ainsi de mesurer le coefficient de frottement pour différentes vitesses d'inclinaison et donc pour différentes vitesses de glissement induites, ce qui n'est pas possible avec le dispositif du document GB 2 455 564.

L'invention a également pour objet un dispositif de détermination d'une force de frottement à l'interface entre deux surfaces en contact l'une avec l'autre, caractérisé en ce que le dispositif comprend :

un support dont l'inclinaison par rapport à l'horizontale est susceptible de varier au cours du temps,

un élément mobile qui est susceptible de glisser sous la seule action de son poids sur le support à partir d'un angle d'inclinaison donné ß dudit support, ledit élément mobile étant conçu de façon à permettre d'appliquer une charge sur deux surfaces en contact et disposées l'une au-dessus de l'autre sur le support, générant ainsi, avec l'inclinaison du support, une force de frottement T à l'interface entre les deux surfaces,

un élément de référence auquel ledit élément mobile est lié par au moins un organe élastique, ledit au moins un organe élastique étant susceptible de se déformer élastiquement selon une direction axiale A et étant apte à exercer une force de retenue sur ledit élément mobile, l'élément de référence ayant une inclinaison qui suit celle du support et une position axiale qui reste fixe quelle que soit l'inclinaison,

des moyens d'augmentation de l'angle d'inclinaison dudit support depuis un état initial dans lequel ledit au moins un organe élastique est dans un état élastiquement prédéformé,

des moyens de mesure de la force de retenue F de l'élément mobile à partir de la déformation élastique dudit au moins un organe élastique,

des moyens de détermination d'une force de frottement à l'interface entre les surfaces en fonction de la force de retenue ainsi mesurée.

Ce dispositif comporte les mêmes avantages et caractéristiques que ceux exposés ci-dessus en relation avec le procédé et ils ne seront donc pas répétés ici.
D'autres caractéristiques et avantages apparaîtront au cours de la description qui va suivre, donnée uniquement à titre d'exemple non limitatif et faite en référence aux dessins annexés, sur lesquels :

- la figure 1 est une vue schématique illustrant la mise en situation de l'invention selon un premier mode de réalisation ;
- la figure 2 représente la mise en situation de l'invention à l'étape initiale du procédé selon l'invention ;
- la figure 3 représente le bilan mécanique des forces appliqué à la figure 1 ;
- la figure 4 est une vue schématique illustrant la mise en situation de l'invention selon un deuxième mode de réalisation.

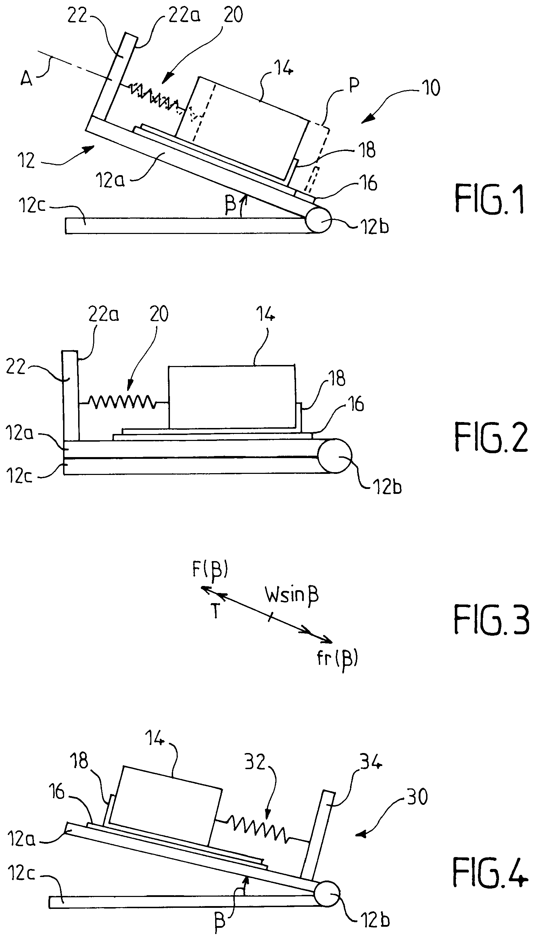

Comme représenté à la figure 1 et désigné par la référence générale notée 10, un dispositif ou appareillage de mise en oeuvre du procédé selon l'invention comprend :

- un support 12 inclinable à partir de 0° (horizontale), par l'intermédiaire d'un mécanisme non représenté suivant une large gamme d'angles d'inclinaison ;
- un élément mobile tel qu'un boîtier mobile 14 posé sur le support 12 et qui est susceptible de glisser sous la seule action de la composante tangentielle de son propre poids à partir d'un certain angle d'inclinaison du support;
- deux couches ou échantillons par exemple de géosynthétiques 16, 18 qui sont respectivement fixés, par exemple par collage (ou par d'autres moyens tels que ceux décrits page 3 de la Norme NF EN ISO 12957-2), à la face supérieure du support 12 et à la face inférieure du boîtier 14 (un géosynthétique est un terme générique désignant un produit, dont au moins l'un des constituants est à base de polymère synthétique ou naturel, qui se présente sous forme de nappe, de bande ou de structure tridimensionnelle, et qui est utilisé en contact avec le sol ou avec d'autres matériaux dans les domaines de la géotechnique et du génie civil ; ce terme est notamment défini en page 3 de la Norme NF EN ISO 10318 (2006) et le terme de sol est notamment défini en page 4 de la Norme NF EN ISO 14688-1 (2003)) de telle façon que la surface supérieure de la couche ou échantillon 16 soit en contact avec la surface inférieure de la couche ou échantillon 18;

- un organe élastique ou élastiquement déformable selon une direction axiale (axe A), tel qu'un ressort 20, qui est fixé d'une part, à une face latérale 14a du boîtier 14 qui est susceptible de se déplacer sur le support en fonction de son angle d'inclinaison et, d'autre part, à une face 22a d'un élément de référence 22 tel qu'une paroi solidaire du support 12 et dont la position axiale reste fixe au cours du mouvement d'inclinaison du support (cet élément de référence suit le mouvement d'inclinaison du support).

Avantageusement, l'organe élastiquement déformable est préalablement déformé, c'est-à-dire qu'il a subi une déformation élastique préalable afin d'être dans un état initial élastiquement prédéformé (ici, il s'agit d'une pré-tension de l'organe 20) avant que le procédé de mesure ne débute et que l'angle d'inclinaison du support ne varie.

Le support 12 à plan ou table inclinable (voir pages 2 et 3 de la Norme NF EN ISO 12957-2 pour plus de précisions) est constitué par exemple d'une plaque 12a lisse et rigide, appelé plaque d'inclinaison, dont une extrémité est dotée d'une charnière 12b. Le support est équipé d'un mécanisme (non représenté) permettant de relever la plaque d'inclinaison à une vitesse constante de par exemple (3,0 + ou -0,5) degrés par minute. Il est également équipé d'un système de mesurage (non représenté) permettant de mesurer l'angle d'inclinaison β (en degrés) formé par la plaque d'inclinaison 12a avec l'embase horizontale 12c.

On notera que boîtier 14 est creux comme défini dans la norme ci-dessus et est disposé sur la couche ou échantillon supérieur 18 de façon à ce qu'il y ait un espace vide de 0,5 mm entre la base du boîtier et la surface supérieure de la couche ou échantillon supérieur 18. Initialement, cet espace libre est créé grâce à des cales. Le réceptacle ou boitier 14 est ensuite rempli d'une masse de sol prédéterminée et d'éventuelles surcharges de poids prédéterminées. Le boîtier repose ainsi sur le sol qui est interposé entre la base dudit boîtier et la surface supérieure de la couche ou échantillon 18 et les cales sont enlevées. Le poids du boitier mobile et du sol le remplissant, additionné du poids d'une éventuelle surcharge, est noté W. Ainsi, une force normale au support est appliquée par cette charge au moment où l'angle β est nul.

Le procédé selon l'invention prévoit d'utiliser par exemple un dispositif ou appareillage 10 selon l'invention tel que décrit précédemment ou dans ses variantes décrites plus loin ou bien dans des variantes non décrites mais qui sont à la portée de l'homme du métier. D'autres dispositifs utilisant le principe de l'invention sont également envisageables.

Le procédé comporte une première phase de mesurage et une deuxième phase de détermination d'une force de frottement à l'interface entre les deux surfaces géosynthétique - géosynthétique 16 et 18 de la figure 1.

On entend par force de frottement à l'interface la force correspondant à la contrainte de cisaillement multipliée par la surface de la face inférieure du boîtier et qui se produit à l'interface entre les deux surfaces des éléments/pièces/échantillons (par exemple de géosynthétiques) en contact l'une avec l'autre.

La première phase de mesurage comporte deux étapes :

1) une première étape qui consiste à appliquer une pré-tension (c'est-à-dire une tension par exemple supérieure ou égale à 1 Newton) dans le ressort 20 servant à retenir le boîtier mobile 14 pour une inclinaison de la plaque d'inclinaison 12a par exemple nulle (état initial de la figure 2) ; cette première étape peut être considérée ou non comme incluse dans la phase de mesurage du procédé ou bien être considérée comme une étape de réglage ou de préparation préalable à la mise en oeuvre de la phase de mesurage du procédé ;

2) une deuxième étape qui consiste à effectuer les opérations suivantes :

- commande de l'inclinaison de la plaque d'inclinaison 12a du support via le mécanisme de relevage afin d'augmenter par exmple régulièrement l'angle d'inclinaison β à partir de l'état initial (fig.2) correspondant à l'horizontale dans cet exemple non limitatif,

- mesure de l'angle d'inclinaison β,

- mesure de la force F(β) nécessaire pour retenir le boîtier mobile 14 pouvant glisser sur la plaque d'inclinaison 12a , quel que soit l'angle d'inclinaison β, c'est-à-dire avant, pendant et après le glissement de l'interface géosynthétique- géosynthétique (glissement du boîtier); cette force F(β) est obtenue soit par mesure directe à l'aide d'un capteur de force placé en série avec le ressort 20, soit en mesurant la déformation du ressort et en utilisant une relation propre au ressort liant la force F(β) et sa déformation. Sur la figure 1 la position P du boîtier mobile 14 après glissement partiel ou total (le ressort 20 s'est étiré de la longueur correspondant au déplacement du boîtier) est représentée en pointillés.

A la suite de la phase de mesurage, la phase de détermination d'une force de frottement à l'interface est mise en oeuvre. Au cours de cette phase on applique une méthode de calcul qui fait intervenir la mesure de la force F(β) en fonction de l'inclinaison β, une équation qui est issue d'un bilan des forces mécaniques (la figure 3 illustre le bilan mécanique des forces exercées dans le dispositif ou appareillage selon l'invention de la figure 1), pour déterminer la force de frottement T à l'interface géosynthétique - géosynthétique. Cette équation s'écrit :T = W sinβ - F(β).

Les différents termes utilisés dans cette équation et illustrés sur la figure 3 sont définis ci-dessous.

W (Newton) : poids du boîtier mobile et du sol remplissant le boîtier mobile additionné du poids d'une éventuelle surcharge.

F(β) (Newton) : force nécessaire pour retenir le boîtier mobile rempli de sol et d'une éventuelle surcharge lorsque la plaque/table d'inclinaison est inclinée d'un angle β.

T (Newton) : force de frottement mobilisée à l'interface géosynthétique - géosynthétique.

La détermination de la force de frottement à l'interface permet ensuite de déterminer d'autres propriétés de frottement à l'interface telles que, par exemple, l'angle de frottement ou le coefficient de frottement.

On notera que l'équation ci-dessus est écrite pour un mouvement de glissement uniforme de l'interface. Une éventuelle force dynamique qui proviendrait d'un mouvement de glissement accéléré de l'interface n'est pas prise en compte car la probabilité d'un mouvement de glissement accéléré de l'interface est réduite par l'utilisation d'un organe élastique initialement prédéformé (ex : ressort ou câble initialement pré-tendu, c'est-à-dire pour une inclinaison du plan inclinable prédéterminée nulle). Toutefois, en cas de succession de mouvements de type « collé-glissé », l'accélération de chaque mouvement unitaire est négligeable et ne remet donc pas en question le principe de l'invention.

Par ailleurs, la condition de mouvement de glissement uniforme de l'interface dépend, pour un dispositif ou appareillage donné, de l'organe élastique utilisé (ex : ressort). Plus l'organe élastique sera souple (faible constante de raideur), plus le mouvement de glissement de l'interface pourra subir une accélération. Inversement, plus l'organe élastique sera raide (grande constante de raideur), plus la condition de mouvement de glissement autorisé de l'interface pourra ne pas être respectée du fait de l'indéformabilité trop importante de l'organe élastique.Ainsi, l'organe élastique (type et/ou raideur) est adapté pour que l'interface, et donc le boîtier, puisse adopter un mouvement de glissement uniforme (vitesse constante).

On notera que le dispositif 10 comprend, pour la mise en oeuvre du procédé, des moyens adaptés pour faire varier l'inclinaison du support 12 (et également la vitesse de variation de l'inclinaison si cela est souhaité), à partir de l'état initial (et pour y revenir), pour mesurer la force de retenue F de l'élément mobile 14 et pour déterminer la force de frottement à l'interface (moyens de calcul).

Selon une variante de réalisation, la vitesse d'inclinaison du support peut varier au cours de la deuxième étape précitée. Par exemple, la vitesse d'augmentation de l'inclinaison du support peut augmenter au cours de cette étape. L'augmentation de cette vitesse induisant une augmentation de la vitesse de glissement, il est ainsi possible de mesure un coefficient de frottement pour différentes vitesses d'inclinaison du support.

Par exemple, cette augmentation de la vitesse d'inclinaison ou de levée du support peut intervenir après que le glissement de l'élément mobile sur le support ait débuté afin que le glissement soit plus rapide. On mesurera ainsi un coefficient de frottement (ou angle de frottement) pour des conditions de glissement dont la vitesse de glissement est contrôlée.

Selon une variante de réalisation, on peut utiliser plusieurs organes élastiques tels que des ressorts montés en série, en parallèle ou suivant un autre montage entre le boîtier et l'élément de référence (paroi).

Selon une autre variante de réalisation non représentée, on détermine une force de frottement à une interface sol-géosynthétique et, dans ce cas, la couche ou échantillon de géosynthétique 18 fixé au boîtier 14 de la figure 1 est supprimé. Le boîtier 14 repose ainsi sur la surface supérieure du géosynthétique inférieur 16 par l'intermédiaire du sol préalablement introduit à l'intérieur du boîtier comme décrit ci-dessus pour le mode de réalisation de la figure 1. La surface inférieure de la masse de sol est ainsi en contact avec la surface supérieure du géosynthétique inférieur 16 et peut glisser sur cette dernière à partir d'un angle d'inclinaison donné du support.

Selon encore une autre variante, le support 12 peut être rempli de sol et le géosynthétique 16 directement posé sur le sol (il n'y a donc pas de plaque 12a mais à la place un bac ouvert vers le haut afin de pouvoir le remplir de sol). Cette variante peut être combinée au mode de réalisation de la figure 1 ou à la variante exposée ci-dessus sans géosynthétique supérieur 18.

Selon une variante supplémentaire, un complexe géosynthétique c'est-à-dire un ou plusieurs géosynthétiques placés entre les géosynthétiques 16 et 18 peut être testé. De façon générale, le complexe géosynthétique comprend plusieurs géosynthétiques disposés les uns au dessus des autres, en contact deux par deux par leurs surfaces en vis-à-vis et formant une structure de type sandwich. Ces géosynthétiques peuvent être libres ou fixés aux géosynthétiques 16 et/ou 18. Dans une telle configuration, les forces de frottement à chaque interface dépendent du comportement de l'ensemble. Dans une telle configuration le ou les organes élastiques jouent bien leur rôle en permettant de mobiliser des forces de frottement croissantes à toutes les interfaces du complexe, tout en autorisant le déplacement des surfaces ou de certaines surfaces les unes par rapport aux autres. Le procédé selon l'invention mis en oeuvre avec une telle configuration permet notamment de déterminer l'interface qui est la plus critique, c'est-à-dire celle qui va glisser la première parmi toutes les interfaces en présence. La phase de calcul exposée ci-dessus est adaptée par l'homme du métier à la présente configuration de type sandwich.

Selon encore une autre variante de réalisation non représentée, le réceptacle ou boitier mobile 14 de la figure 1 est muni de roulettes et repose sur un système de guidage de type rails solidaire du support 12 tel que décrit dans la norme mentionnée ci-dessus, notamment au chapitre 7 relatif à l'appareillage. Dans cette variante, le poids du sol remplissant le boîtier s'exerce directement sur les deux surfaces en regard des deux géosynthétiques en contact.

Dans cette variante, l'équation ci-dessus s'écrit :

$$ T = W \sin\beta + fr(\beta) - F(\beta) $$

où les différents termes utilisés sont représentés sur la figure 3 ont la signification suivante (les termes β et T ont la même définition que celle donnée plus haut):

W (Newton) : poids du sol remplissant le boîtier additionné du poids d'une éventuelle surcharge et du poids des parties du boîtier non soutenues par les roulettes.

F(β) (Newton) : force nécessaire pour retenir le boîtier mobile rempli de sol et d'une éventuelle surcharge lorsque la plaque/table d'inclinaison est inclinée d'un angle β.

fur(β) (Newton) : force nécessaire pour retenir le boîtier mobile vide lorsque la plaque /table d'inclinaison est inclinée d'un angle β.

La détermination de la force de frottement est réalisée comme décrit ci-dessus pour le premier mode de réalisation de la figure 1. Seul le calcul de la force de frottement à l'interface géosynthétique - géosynthétique diffère du fait de l'équation modifiée ci-dessus.

Dans une variante additionnelle, l'élément mobile est un objet solide tel qu'un bloc rigide dont le poids va s'exercer directement sur les deux surfaces en regard des deux géosynthétiques en contact, voire des différentes surfaces pour un géosynthétique complexe.

Un deuxième mode de réalisation de dispositif 30 est illustré à la figure 4 et reprend les mêmes éléments que ceux de la figure 1 qui conservent leur référence, hormis le fait que l'organe élastique 32 (ressort, câble...) ne retient plus le boîtier mobile 14 par le haut mais par le bas.

Plus particulièrement, l'organe élastiquement déformable 32 est prédéformé en compression (précompression) et non plus en tension, tendant ainsi à empêcher le boîtier 14 de se rapprocher de l'élément d'attache ou de référence 34 (ex : paroi) disposé en partie basse, par exemple à proximité de l'articulation 12b du support. On notera que tout ce qui a été dit précédemment à propos du premier mode de réalisation et de ses variantes, y compris les équations ci-dessus reste valable.

Selon une variante non représentée, les modes de réalisation des figures 1 et 4 sont combinés de telle façon qu'un organe élastique est disposé de part et d'autre du boîtier mobile 14 pour, d'un côté, retenir le boîtier par un effort d'extension (agencement de la figure 1) et, du côté opposé, le retenir par un effort de compression. La force de retenue du boîtier qui est mesurée peut être l'une ou l'autre force qui est associée à l'organe 20 ou à l'organe 32 ou une force dérivée de la combinaison des deux forces mesurées ex : moyenne).

A titre de variantes de réalisation, les modes de réalisation décrits ci-dessus et leurs variantes s'appliquent également à des surfaces en contact qui ne sont pas nécessairement des surfaces géosynthétiques.

Ainsi, le procédé de détermination d'une force de frottement à l'interface entre deux surfaces en contact l'une avec l'autre peut s'appliquer, de manière non limitative, à :

- deux surfaces roche/roche (ex : application intéressante entre des joints rocheux) ;
- deux surfaces béton/béton (ex : application intéressante pour évaluer le frottement entre deux surfaces en béton dans le cas d'une fissure) ;
- deux surfaces sol/béton (ex : application intéressante pour évaluer la stabilité d'une dalle en béton sur un sol en pente) ;
- deux surfaces sol/roche.

## Revendications

1. Procédé de détermination d'une force de frottement à l'interface entre deux surfaces en contact l'une avec l'autre, le procédé mettant en oeuvre un support (12) dont l'inclinaison par rapport à l'horizontale est susceptible de varier au cours du temps et un élément mobile (14) qui est susceptible de glisser sur le support sous la seule action de son poids à partir d'un angle d'inclinaison donné β dudit support, ledit élément mobile étant conçu de façon à permettre d'appliquer une charge sur deux surfaces (16,18) en contact et disposées l'une au dessus de l'autre sur le support, générant ainsi, avec l'inclinaison du support, une force de frottement (T) à l'interface entre les deux surfaces, ledit élément mobile étant lié à un élément de référence (22 ;34) par au moins un organe élastique (20 ;32) qui est susceptible de se déformer élastiquement selon une direction axiale (A) et qui est apte à exercer une force de retenue sur ledit élément mobile (14), l'élément de référence ayant une inclinaison qui suit celle du support et une position axiale qui reste fixe quelle que soit l'inclinaison, le procédé comprenant les phases suivantes :

    - augmentation de l'angle d'inclinaison dudit support depuis un état initial dans lequel ledit au moins un organe élastique (20 ; 32) est dans un état élastiquement prédéformé, dans l'état initial ledit élément mobile n'ayant pas commencé à glisser et ledit angle d'inclinaison du support n'ayant pas encore été modifié,

- mesure de la force de retenue (F) de l'élément mobile (14) à partir de la déformation élastique dudit au moins un organe élastique,

- détermination d'une force de frottement à l'interface entre les surfaces en fonction de la force de retenue ainsi mesurée.

2. Procédé selon la revendication 1, **caractérisé en ce que** ledit au moins un organe élastiquement déformable (20 ; 32) est prédéformé suivant une force supérieure ou égale à 1 N.

3. Procédé selon la revendication 1 ou 2, **caractérisé en ce que** dans l'état initial l'angle d'inclinaison du support (12) dépend de la nature des surfaces en contact.

4. Procédé selon l'une des revendications 1 à 3, **caractérisé en ce que** ledit au moins un organe élastique est un ressort (20 ; 32) ou un câble élastique.

5. Procédé selon l'une des revendications 1 à 4, **caractérisé en ce que** l'élément mobile est un boîtier (14).

6. Procédé selon la revendication 5, **caractérisé en ce que** le boîtier est préalablement rempli d'un matériau de remplissage d'un poids prédéterminé.

7. Procédé selon l'une des revendications 1 à 6, **caractérisé en ce que** le procédé prévoit de mesurer la force de retenue avant que l'élément mobile (14) n'adopte un mouvement de glissement sur le support (12) à partir d'un angle d'inclinaison donné dudit support.

8. Procédé selon l'une des revendications 1 à 7, **caractérisé en ce que** à partir d'un angle d'inclinaison donné l'élément mobile (14) adopte un mouvement de glissement sur le support (12) et ledit au moins un organe élastique se déforme élastiquement au fur et à mesure que l'angle d'inclinaison augmente, la force de retenue étant mesurée durant ledit mouvement de glissement.

9. Procédé selon l'une des revendications 1 à 8, **caractérisé en ce que** l'élément de référence (22 ; 34) est fixé au support (12).

10. Procédé selon l'une des revendications 1 à 9, **caractérisé en ce que** ledit au moins un organe élastique (20) est prédéformé en étirement et tend à empêcher l'élément mobile (14) de s'éloigner de l'élément de référence (22).

11. Procédé selon l'une des revendications 1 à 9, **caractérisé en ce que** ledit au moins un organe élastique (32) est prédéformé en compression et tend à empêcher l'élément mobile (14) de se rapprocher de l'élément de référence (34).

12. Procédé selon l'une des revendications 1 à 11, **caractérisé en ce qu'**au moins une des deux surfaces en contact l'une avec l'autre est une surface de type géosynthétique.

13. Procédé selon l'une des revendications 1 à 12, **caractérisé en ce que** l'augmentation de l'angle d'inclinaison dudit support est effectuée avec une vitesse variable.

14. Dispositif de détermination d'une force de frottement à l'interface entre deux surfaces en contact l'une avec l'autre, **caractérisé en ce que** le dispositif comprend :

- un support (12) dont l'inclinaison par rapport à l'horizontale est susceptible de varier au cours du temps à partir d'un état initial,

- un élément mobile (14) qui est susceptible de glisser sous la seule action de son poids sur le support à partir d'un angle d'inclinaison donné ß dudit support, ledit élément mobile étant conçu de façon à permettre d'appliquer une charge sur deux surfaces (16, 18) en contact et disposées l'une au-dessus de l'autre sur le support, générant ainsi, avec l'inclinaison du support, une force de frottement (T) à l'interface entre les deux surfaces,

- un élément de référence (22 ; 34) auquel ledit élément mobile (14) est lié par au moins un organe élastique (20 ; 32), ledit au moins un organe élastique (20 ; 32) étant susceptible de se déformer élastiquement selon une direction axiale (A) et étant apte à exercer une force de retenue sur ledit élément mobile (14), l'élément de référence ayant une inclinaison qui suit celle du support et une position axiale qui reste fixe quelle que soit l'inclinaison,

- des moyens d'augmentation de l'angle d'inclinaison dudit support depuis l'état initial dans lequel ledit au moins un organe élastique (20 ; 32) est dans un état élastiquement prédéformé, dans l'état initial ledit élément mobile n'ayant pas commencé à glisser et ledit angle d'inclinaison du support n'ayant pas encore été modifié,

- des moyens de mesure de la force de retenue (F) de l'élément mobile (14) à partir de la déformation élastique dudit au moins un organe élastique,

- des moyens de détermination d'une force de frottement à l'interface entre les surfaces en fonction de la force de retenue ainsi mesurée.

## Patentansprüche

1. Verfahren zur Herstellung einer Reibungskraft an der Schnittstelle zwischen zwei Flächen in Kontakt miteinander, wobei das Verfahren einen Träger (12) implementiert, dessen Neigung mit Bezug auf die Horizontale im Laufe der Zeit variieren kann, und ein bewegliches Element (14), das auf dem Träger nur unter der Wirkung seines Gewichts ab einem gegebenen Neigungswinkel β des Trägers gleiten kann, wobei das bewegliche Element derart entworfen ist, dass es ermöglicht, eine Last auf zwei Flächen (16, 18) in Kontakt und angeordnet aufeinander auf dem Träger anzuwenden, wodurch, mit der Neigung des Trägers, eine Reibungskraft (T) an der Schnittstelle zwischen den zwei Flächen erzeugt wird, wobei das bewegliche Element an ein Referenzelement (22, 34) durch mindestens ein elastisches Organ (20, 32) verbunden ist, das sich gemäß einer axialen Richtung (A) elastisch verformen kann, und das ausgelegt ist, um eine Rückhaltekraft auf das bewegliche Element (14) auszuüben, wobei das Referenzelement eine Neigung aufweist, die derjenigen des Trägers folgt, und eine axiale Position, die fixiert bleibt, unabhängig von der Neigung, wobei das Verfahren die folgenden Schritte umfasst:

    - Erhöhen des Neigungswinkels des Trägers von einem anfänglichen Zustand, in dem sich das mindestens eine elastische Organ (20, 32) in einem elastisch vorverformten Zustand befindet, wobei im anfänglichen Zustand das bewegliche Element nicht angefangen hat, zu gleiten, und der Neigungswinkel des Trägers noch nicht modifiziert wurde,
    - Messen der Rückhaltekraft (F) des beweglichen Elements (14) von der elastischen Verformung des mindestens einen elastischen Elements,
    - Bestimmen einer Reibungskraft an der Schnittstelle zwischen den Flächen in Funktion der so gemessenen Rückhaltekraft.

2. Verfahren nach Anspruch 1, **dadurch gekennzeichnet, dass** das mindestens eine elastisch verformbare Organ (20, 32) gemäß einer Kraft von mehr als oder gleich 1 N vorverformt ist.

3. Verfahren nach Anspruch 1 oder 2, **dadurch gekennzeichnet, dass** im anfänglichen Zustand der Neigungswinkel des Trägers (12) von der Art der Flächen in Kontakt abhängt.

4. Verfahren nach einem der Ansprüche 1 bis 3, **dadurch gekennzeichnet, dass** das mindestens eine elastische Organ eine Feder (20; 32) oder ein elastisches Kabel ist.

5. Verfahren nach einem der Ansprüche 1 bis 4, **dadurch gekennzeichnet, dass** das bewegliche Element ein Gehäuse (14) ist.

6. Verfahren nach Anspruch 5, **dadurch gekennzeichnet, dass** das Gehäuse im Voraus mit einem Füllmaterial mit einem vorbestimmten Gewicht gefüllt wird.

7. Verfahren nach einem der Ansprüche 1 bis 6, **dadurch gekennzeichnet, dass** das Verfahren vorsieht, die Rückhaltekraft zu messen, bevor das bewegliche Element (14) eine Gleitbewegung auf dem Träger (12) ab eine gegebenen Neigungswinkel β des Trägers beginnt.

8. Verfahren nach einem der Ansprüche 1 bis 7, **dadurch gekennzeichnet, dass** ab einem gegebenen Neigungswinkel das bewegliche Element (14) eine Gleitbewegung auf dem Träger (12) beginnt, und das mindestens eine elastisch verformbare Organ sich elastisch verformt, sobald sich der Neigungswinkel erhöht, wobei die Rückhaltekraft während der Gleitbewegung gemessen wird.

9. Verfahren nach einem der Ansprüche 1 bis 8, **dadurch gekennzeichnet, dass** das Referenzelement (22, 34) an den Träger (12) fixiert ist.

10. Verfahren nach einem der Ansprüche 1 bis 9, **dadurch gekennzeichnet, dass** das mindestens eine elastische Organ (20) durch Dehnung vorverformt wird und dazu neigt, das bewegliche Element (14) daran zu hindern, sich vom Referenzelement (22) zu entfernen.

11. Verfahren nach einem der Ansprüche 1 bis 9, **dadurch gekennzeichnet, dass** das mindestens eine elastische Organ (32) durch Dehnung vorverformt ist und dazu neigt, das bewegliche Element (14) daran zu hindern, sich dem Referenzelement (34) zu nähern.

12. Verfahren nach einem der Ansprüche 1 bis 11, **dadurch gekennzeichnet, dass** mindestens eine der zwei Flächen in Kontakt miteinander eine Fläche von geosynthetischer Art ist.

13. Verfahren nach einem der Ansprüche 1 bis 12, **dadurch gekennzeichnet, dass** die Erhöhung des Neigungswinkels des Trägers mit einer variablen Geschwindigkeit erfolgt.

14. Vorrichtung zur Bestimmung einer Reibungskraft an der Schnittstelle zwischen zwei Flächen in Kontakt miteinander, **dadurch gekennzeichnet, dass** die Vorrichtung Folgendes umfasst:

- einen Träger (12), dessen Neigung mit Bezug auf die Horizontale im Laufe der Zeit von einem anfänglichen Zustand variieren kann,

- ein bewegliches Element (14), das nur unter der Wirkung seines Gewichts auf dem Träger ab einem gegebenen Neigungswinkel $\beta$ des Trägers gleiten kann, wobei das bewegliche Element derart entworfen ist, dass es ermöglicht, eine Last auf zwei Flächen (16, 18) in Kontakt und angeordnet aufeinander auf dem Träger anzuwenden, wodurch, mit der Neigung des Trägers, eine Reibungskraft (T) an der Schnittstelle zwischen den zwei Flächen erzeugt wird,

- ein Referenzelement (22; 34), mit dem das bewegliche Element (14) durch mindestens ein elastisches Organ (20; 32) verbunden ist, wobei das mindestens eine elastische Organ (20; 32) sich gemäß einer axialen Richtung (A) elastisch verformen kann und ausgelegt ist, um eine Rückhaltekraft auf das bewegliche Element (14) auszuüben, wobei das Referenzelement eine Neigung aufweist, die derjenigen des Trägers folgt, und eine axiale Position, die fixiert bleibt, unabhängig von der Neigung,

- und Mittel zur Erhöhung des Neigungswinkels des Trägers von einem anfänglichen Zustand, in dem sich das mindestens eine elastische Organ (20, 32) in einem elastisch vorverformten Zustand befindet, wobei im anfänglichen Zustand das bewegliche Element nicht mit dem Gleiten begonnen hat und der Neigungswinkel des Trägers noch nicht modifiziert wurde,

- Mittel zum Messen der Rückhaltekraft (F) des beweglichen Elements (14) von der elastischen Verformung des mindestens einen elastischen Elements,

- Mittel zum Bestimmen einer Reibungskraft an der Schnittstelle zwischen den Flächen in Funktion der so gemessenen Rückhaltekraft.

**Claims**

1. A method for determining a friction force at the interface between two surfaces in contact with one another, the method implementing a support (12) whose incline relative to the horizontal may vary over time and a moving element (14) that may slide on the support under the sole action of its weight from a given incline angle ß of said support, said moving element being designed so as to make it possible to apply a load on two surfaces (16, 18) in contact and arranged one above the other on the support, thereby generating, with the incline of the support, a friction force (T) at the interface between the two surfaces, said moving element being connected to a reference element (22; 34) by at least one elastic member (20; 32) that is able to deform elastically along a direction (A) and that is able to exert a retaining force on said moving element (14), the reference element having an incline that follows that of the support and an axial position that remains fixed irrespective of the incline, the method comprising the following steps:

   - increasing the incline angle of said support from an initial state in which said at least one elastic member (20; 32) is in an elastically pre-deformed state, in the initial state of moving element not having begun to slide and said incline angle of the support not yet having been modified,

   - measuring the retaining force (F) of the moving element (14) from the elastic deformation of said at least one elastic member,

   - determining a friction force at the interface between the surfaces based on the retaining force thus measured.

2. The method according to claim 1, **characterized in that** said at least one elastically deformable member (20; 32) is pre-deformed with a force greater than or equal to 1 N.

3. The method according to claim 1 or 2, **characterized in that** in the initial state, the incline angle of the support (12) depends on the nature of the surfaces in contact.

4. The method according to one of claims 1 to 3, **characterized in that** said at least one elastic member is a spring (20; 32) or an elastic cable.

5. The method according to one of claims 1 to 4, **characterized in that** the moving element is a casing (14).

6. The method according to claim 5, **characterized in that** the casing is filled beforehand with a filler material having a predetermined weight.

7. The method according to one of claims 1 to 6, **characterized in that** the method provides for measuring the retaining force before the moving element (14) adopts a sliding movement on the support (12) for a given incline angle of said support.

8. The method according to one of claims 1 to 7, **characterized in that** from a given incline angle, the moving element (14) adopts a sliding movement on the support (12) and said at least one elastic member deforms elastically as the incline angle increases, the retaining force being measured during said sliding movement.

9. The method according to one of claims 1 to 8, **char-**

**acterized in that** the reference element (22; 34) is fixed to the support (12).

10. The method according to one of claims 1 to 9, **characterized in that** said at least one elastic member (20) is pre-deformed by stretching and tends to prevent the moving element (14) from moving away from the reference element (22).

11. The method according to one of claims 1 to 9, **characterized in that** said at least one elastic member (32) is pre-deformed by compression and tends to prevent the moving element (14) from coming closer to the reference element (34).

12. The method according to one of claims 1 to 11, **characterized in that** at least one of the two surfaces in contact with one another is a surface of the geosynthetic type.

13. The method according to one of claims 1 to 12, **characterized in that** the increase of the incline angle of the support is done with a variable speed.

14. A device for determining a friction force at the interface between two surfaces in contact with one another, **characterized in that** the device comprises:

- a support (12) whose incline relative to the horizontal may vary over time from an initial state,
- a moving element (14) is able to slide under the sole action of its weight on the support from a given incline angle ß of said support, said moving element being designed so as to make it possible to apply a load on two surfaces (16, 18) in contact and arranged one above the other on the support, thereby generating, with the incline of the support, a friction force T at the interface between the two surfaces,
- a reference element (22; 34) to which said moving element (14) is connected by at least one elastic member (20; 32), said at least one elastic member (20; 32) being able to deform elastically along an axial direction (A) and being able to exert a retaining force on said moving element (14), the reference element having an incline that follows that of the support and an axial position that remains fixed irrespective of the incline,
- means for increasing the incline angle of said support from the initial state in which said at least one elastic member (20; 32) is in an elastically pre-deformed state, in the initial state of the moving element not having begun to slide and said incline angle of the support not yet having been modified,
- means for measuring the retaining force (F) of the moving element (14) from the elastic deformation of said at least one elastic member,
- means for determining a friction force at the interface between the surfaces based on the retaining force thus measured.

FIG.1

FIG.2

$$F(\beta)$$
$$T \quad Wsin\beta$$
$$fr(\beta)$$

FIG.3

FIG.4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

• GB 2455564 A

**Littérature non-brevet citée dans la description**

• **DE BRIANÇON et al.** Justification d'une modification de la norme relative au frottement des interfaces géo-synthétiques au plan incliné. *Rencontres Géosynthétiques,* 2011, 219-228